# EUROPEAN PATENT SPECIFICATION

(11) **EP 4 031 198 B1**
(45) Date of publication and mention of the grant of the patent: **18.11.2026**
(21) Application number: 20781100.1
(22) Date of filing: 19.09.2020
(51) Int. Cl.: A61L 2/20, A61L 2/206, A61L 2/208, A61L 2/28, C12M 1/12, C12Q 1/22

(54) **BIOLOGICAL INDICATOR FOR USE WITH A LIQUID STERILANT**
BIOLOGISCHER INDIKATOR ZUR VERWENDUNG MIT EINEM FLÜSSIGEN STERILISIERUNGSMITTEL
INDICATEUR BIOLOGIQUE DESTINÉ À ÊTRE UTILISÉ AVEC UN AGENT STÉRILISANT LIQUIDE

(30) Priority: 20.09.2019 US 201962903540 P
(43) Date of publication of application: 27.07.2022
(73) Proprietor: ASP Global Manufacturing GmbH, 8207 Schaffhausen (CH)
(72) Inventor: TRUONG, Doug V., Irvine, California 92618 (US); RHODES, Sam J., Irvine, California 92618 (US); OMIDBAKHSH, Navid, Irvine, California 92618 (US)
(74) Representative: Regimbeau
(86) International application number: PCT/IB2020/058750
(87) International publication number: WO 2021/053627

(56) References cited:
- WO-A1-2016/057520
- WO-A1-2018/160449
- WO-A1-2020/136463
- WO-A2-2010/039388
- WO-A2-2010/045138
- CN-A- 105 561 362
- US-A- 5 770 393
- US-A1- 2017 253 845
- US-A1- 2018 237 821

## Description

### FIELD

The subject matter disclosed herein relates to biological indicators, particularly those suitable for use in liquid-chemical decontamination systems and procedures for disinfection or sterilization.

### BACKGROUND

Certain instruments, e.g., medical instruments, should be reprocessed, i.e., decontaminated, between medical procedures in which they are used to avoid causing infection or illness in a subject. Two decontamination methods pertinent to the present disclosed subject matter include disinfection and liquid-chemical sterilization. Both types of procedures may include steps of removing foreign material from the endoscope, cleaning the endoscope, introducing a disinfectant solution or a liquid-chemical sterilant to the endoscope, rinsing the endoscope, and drying the endoscope

A decontamination indicator is a device that may be placed alongside or in proximity to a medical device being subject to a decontamination procedure, such that the decontamination indicator is subject to the same decontamination cycle as the medical device. For instance, a biological indictor having a predetermined quantity of microorganisms possessing known resistance to the sterilant may be placed into a sterilization chamber alongside a medical device and subjected to a sterilization cycle. After the sterilization procedure is complete, the microorganisms in the biological indicator may be cultured to determine whether any of the microorganisms survive.

Biological indicators often include a housing that contains a quantity of microorganisms and a source of growth media in a frangible container that is located near the microorganisms. Following a sterilization procedure, the frangible container may be broken to release the growth media and culture any surviving microorganisms in situ. The indicator may then be incubated at elevated temperatures, typically around 50°C to 60°C, which encourages outgrowth of the surviving microorganisms.

Patent document WO2010/039388 discloses a self-contained sterilization indicator for evaluating the effectiveness of a sterilization process. Patent document WO2018/160449 discloses a recovery medium for detection of microorganisms by fluorescence. Patent document US2018/237821 discloses a method for detecting biological activity in a biological indicator. Patent document WO2020/136463 discloses a method for indication of treatment. Patent application WO2010/045138 discloses a biological sterilization indicator, system, and methods of determining the effectiveness of a sterilization process. Patent application US2017/253845 discloses a biological sterilization indicator comprising a housing having a first enclosure and a second enclosure, an ampule containing a liquid growth medium, and an insert disposed at least partially in the first enclosure. However, the biological sterilization indicator of this patent application does allow the displacement of air from inside the housing by any liquids introduced thereto. Patent CN105561362 discloses a rapid biology sterilization detection tube device, which comprises a liquid containing tube for containing a culture solution and a culture tube for containing a thallus carrier.

The frangible container, e.g., ampule, that contains the liquid growth medium is often fabricated from glass. The glass must be sufficiently robust to avoid breakage during transportation, e.g., from the manufacturer of the biological indicator to a health care provider. Such robustness, however, corresponds to a greater force required to break the ampule at the desired time by medical personnel. Accordingly, some manufacturers provide activation devices to hospital personnel to assist them in breaking the ampule.

### SUMMARY OF THE DISCLOSURE

The invention relates to a biological indicator according to claim 1. The sidewall may include a round portion and a flat portion, the sidewall of the second portion defining a side boundary of the liquid chamber.

The insert may have a tip portion, e.g., a spike, disposed along the longitudinal axis, for puncturing the seal. As such, an ampule containing a growth medium is not disposed in the housing.

### BRIEF DESCRIPTION OF THE DRAWINGS

While the specification concludes with claims, which particularly point out and distinctly claim the subject matter described herein, it is believed the subject matter will be better understood from the following description of certain examples taken in conjunction with the accompanying drawings, in which like reference numerals identify the same elements and in which:
Figure 1 depicts a front view of a biological indicator;
Figure 2 depicts a side view of the biological indicator;
Figure 3 depicts a perspective view of the biological indicator;
Figure 4 depicts an exploded view of the biological indicator; and
Figure 5 depicts a cross-sectional view of a cap of the biological indicator taken along line 5-5 in Figure 4.

### MODES OF CARRYING OUT THE INVENTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment.

Commercially available biological indicators are typically used in sterilization procedures that utilize gaseous sterilants, e.g., ethylene oxide and hydrogen peroxide. Such procedures often involve positioning medical devices alongside a sterilization indicator in a vacuum chamber of a sterilization system. After a vacuum (e.g., less than five torr) is drawn in the chamber, the gaseous sterilant may be introduced into the chamber, which raises the pressure in the chamber, such that gaseous sterilant will enter into the biological indicator. Subsequently, the gaseous sterilant is removed from the biological indicator and the chamber by drawing a vacuum, venting the chamber, or both.

A biological indicator suitable for use in decontamination procedures that utilize liquid-chemical decontaminants is disclosed. Such decontamination procedures may include disinfection procedures that utilize a liquid-chemical disinfectant, e.g., a solution comprising ortho-Phthalaldehyde, and sterilization procedures that utilize liquid-chemical sterilants, e.g., a solution comprising peracetic acid. As such, ortho-Phthalaldehyde solutions and peracetic acid solutions may be considered examples of liquid-chemical decontaminants or decontaminant solutions. During a decontamination procedure, but after an instrument and a biological indicator have been exposed to a liquid decontaminant for a suitable amount of time, the liquid decontaminant must be removed from the instrument and the biological indicator. There are various reasons that the liquid decontaminant must be removed from the biological indicator. For example, removal helps avoid injury to healthcare personnel that might be caused by skin contact with the decontaminant. Removal of the decontaminant also enables an accurate assessment of microbial outgrowth in the biological indicator after the decontamination procedure has ended.

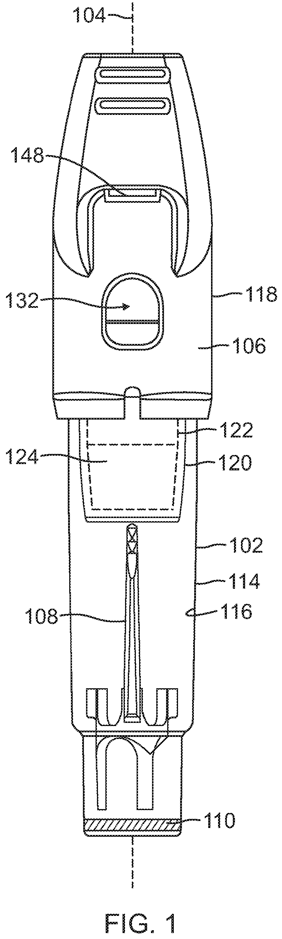

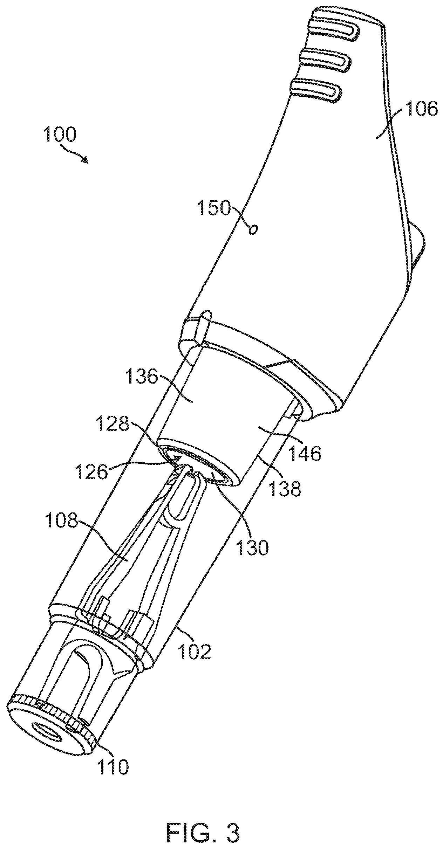

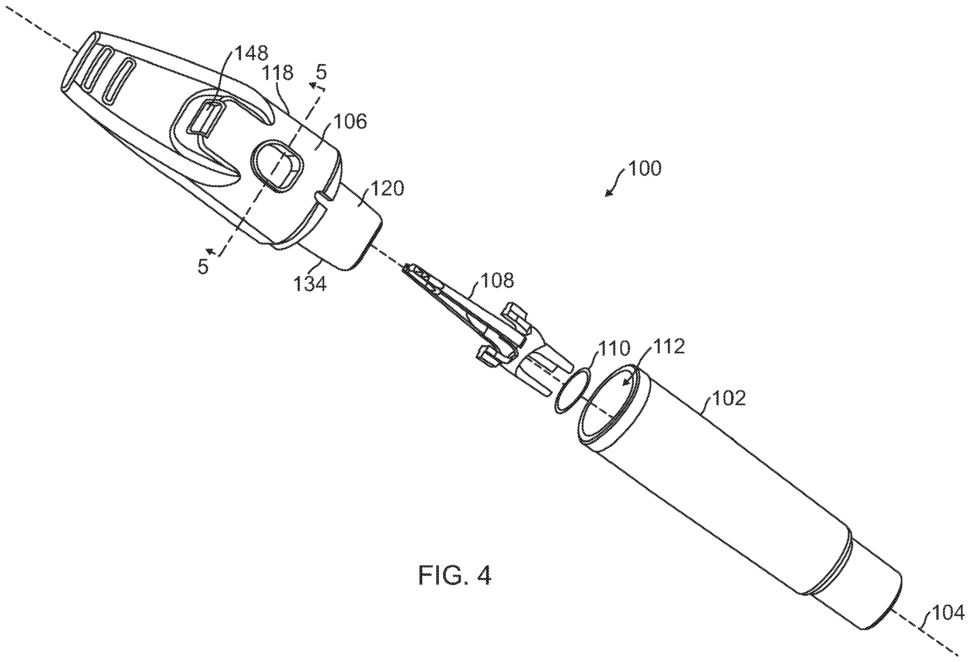

Figures 1-4 reflect a biological indicator 100 that is suitable for use in a decontamination procedure that utilizes a liquid decontaminant. Biological indicator 100 includes a housing 102 defining a longitudinal axis 104 of biological indicator 100, a cap 106, an insert 108, and a source of microorganisms or active enzymes, such as carrier 110.

Housing 102 may have an elongate cylindrical form and includes a first port 112, an outside surface 114, and an inside surface 116. Cap 106 is coupled to housing 102 such that cap 106 covers first port 112.

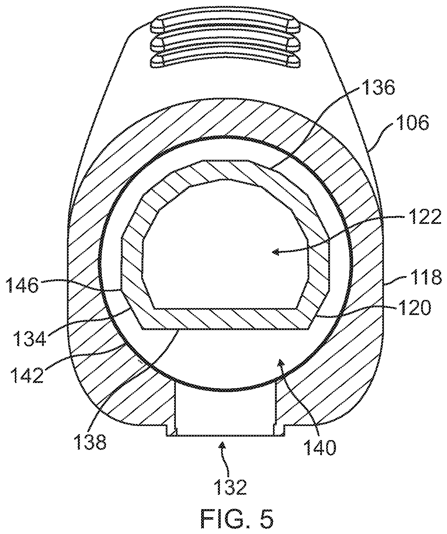

With additional reference to Figure 5, cap 106 comprises a first portion 118 and a second portion 120 that define an annular recess 140 therebetween. A top portion of housing 102 that includes first port 112 may be disposed in recess 140 such that cap 106 covers first port 112. As such an inner surface 142 of first portion 118 may contact outer surface 114 of housing 102, thus coupling housing 102 and cap 106 by a friction fit. For example, inner surface 142 and outer surface 114 may each have diameters of between about 10 millimeters and 15 millimeters, provided that the diameter of inner surface 142 is slightly less than the diameter of outer surface 114, e.g., about 0.1 millimeters and about 2 millimeters less. For example, inner surface 142 may have a diameter of about 12.5 millimeters and outer surface 114 may have a diameter of about 12.6 millimeters.

Notably, biological indicator 100 lacks an ampule containing a growth medium, which the inventors have found impedes introduction and removal of liquids (e.g., decontaminant, neutralizer) into and from housing 102. As such, the inventors designed biological indicator 100 such that second portion 120 of cap 106 includes a liquid chamber 122 containing a growth medium 124. Liquid chamber 122 includes a second port 126 covered by a seal 128 such that seal 128 defines a bottom boundary 130 of liquid chamber 122. As cap 106 is coupled to housing 102, second portion 120 is disposed at least partially inside housing 102 such that seal 128 and bottom boundary 130 of liquid chamber 122 are disposed inside housing 102. Second portion 120 also includes a sidewall 134 that forms a side boundary 146 of liquid chamber 122. Sidewall 134 may comprise a round portion 136 and a flat portion 138. Accordingly, recess 140 between first portion 118 and second portion 120 is larger proximate to flat portion 138 than proximate to round portion 136. For example, the distance between inside surface 116 of housing 102 and round portion 136 of sidewall 134 may be between approximately 0.1 millimeters and approximately 0.2 millimeters, whereas the distance between inside surface 116 of housing 102 and flat portion 138 of sidewall 134 may be between approximately 1 millimeter and approximately 3 millimeters. For example, the distance between inside surface 116 of housing 102 and round portion 136 of sidewall 134 may be approximately 0.13 millimeters, whereas the distance between inside surface 116 of housing 102 and flat portion 138 of sidewall 134 may be approximately 2 millimeters.

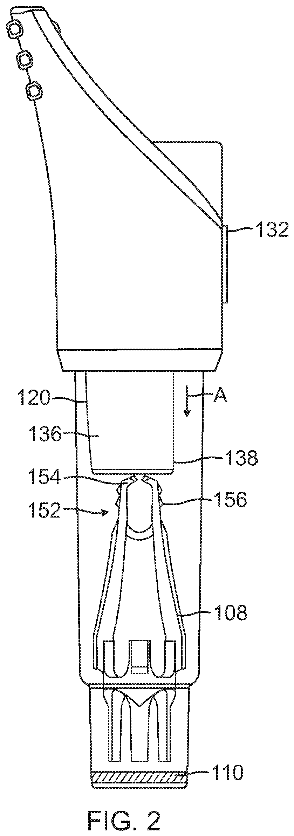

First portion 118 of cap 106 comprises a third port 132 that provides access to recess 140. As such, as cap 106 is coupled to housing 102, third port 132 provides access to the inside of housing 102 via port 112 and a liquid passage that extends through a space disposed between sidewall 134, preferably flat portion 138 of sidewall 134, and inside surface 116 of housing 102. Arrow A is shown in Figure 2 in this liquid passage to indicate the direction that liquid introduced through third port 132 would flow into housing 102.

First portion 118 of cap 106 may additionally include a detent 148, which may be useful for positioning biological indicator 100 in a decontamination system. That is, detent 148 may mate with a corresponding mating feature of the decontamination system such that third port 132 is positioned optimally for being mated with any tubing or flow connectors that may be used to deliver and withdraw liquids, e.g., decontaminants or neutralizers, into and out of housing 102. Because liquids introduced into housing 102 must be withdrawn therefrom to enable an accurate assessment of microbial outgrowth following the procedure, removal of liquids may be facilitated when biological indicator 100 is in a substantially horizontal position, i.e., substantially perpendicular to gravitational forces, with third port 132 facing down. A vent 150 may also be provided in cap 106, preferably in first portion 118. Vent 150 permits displacement of air from inside housing 102 by any liquids introduced thereto. Vent 150 also facilitates removal of liquids from inside housing 102, by allowing air to reenter housing 102 as the liquids are withdrawn.

Insert 108 is provided for two reasons. First, it helps maintain the position of carrier 110 at the bottom of housing 102. Second, it is used to pierce seal 128, which allows growth medium 124 to flow out of liquid chamber 122 and into housing 102 to immerse carrier 110. As best seen in Figure 2, insert 108 includes a tip portion 152. Tip portion 152 may have the form of a spike, which may be forked, e.g., including two or more tines 154. Insert 108 may be fabricated of a suitable material, e.g., a polymer such as Cyclo-Olefin Polymer, and with tines 154 having a suitable form, e.g., a thickness of between about 0.2 mm and about 1 mm, e.g., about 0.5 mm, such that tines 154 may flex when subject to downward pressure. As such, when cap 106 is depressed downward, tip portion 152 pierces seal 128, which may be a film, such as a foil, e.g., aluminum foil. Upon initial contact between seal 128 and tip portion 152, tines 154 flex toward each other before the seal becomes punctured. Once seal 128 is punctured, tines 154 revert to their original position, thus enlarging the hole created in seal 128. Each tine 154 may additionally include surface features, e.g., ribs 156, which may further enlarge the hole in seal 128 as seal 128 moves downward over them.

By virtue of the embodiments illustrated and described herein, Applicant has devised a method and variations thereof for using a biological indicator of having those features described hereinabove. First, the biological indicator may be received by a user, e.g., by healthcare personnel. Then, the biological indicator may be disposed in a liquid-chemical decontamination system. In some variations, the biological indicator may be positioned in the chamber in a horizontal position. In further variations, it is positioned so the third port faces down. Next, the liquid decontaminant may be flowed into the housing via the third port in the cap. Subsequently, the liquid decontaminant may be removed from the housing via the third port in the cap. After the decontamination procedure is complete, the biological indicator may be removed from the chamber. Then, the cap may be depressed such that the seal becomes punctured by the top portion of the insert being driven through the seal, causing the growth medium in the liquid chamber of the cap to flow into the housing, through the puncture in the seal, to submerge the carrier. Simultaneously, vent 150 and third port 132 become blocked by housing 102.

Any of the examples or embodiments described herein may include various other features in addition to or in lieu of those described above. The teachings, expressions, embodiments, examples, etc., described herein should not be viewed in isolation relative to each other. Various suitable ways in which the teachings herein may be combined should be clear to those skilled in the art in view of the teachings herein, as long as they remain within the scope of the claims.

## Claims

1. A biological indicator (100), comprising:
a housing (102) defining a longitudinal axis (104), the housing (102) having a first port (112);
a cap (106) coupled to the housing (102), the cap (106) comprising,
a vent (150),
a first portion (118) disposed outside the housing (102), the first portion comprising a third port disposed through the first portion,
a second portion (120) disposed at least partially inside the housing (102), the second portion (120) including a liquid chamber (122) comprising a second port (126) and containing a growth medium (124), and a seal (128) covering the second port (126) to define a bottom boundary (130) of the liquid chamber (122), and
an insert (108) disposed in the housing (102), the insert (108) having a tip portion (152) disposed along the longitudinal axis (104), in which the second portion (120) of the cap (106) comprises a sidewall (134) defining a side boundary 146) of the liquid chamber (122) such that the second port (126) is disposed in the housing (102) about the longitudinal axis (104) of the housing (102),
further comprising a liquid passage that extends through a space disposed between the sidewall (134) of the second portion (120) of the cap (106) and an inside surface (116) of the housing (102), the third port (132) providing access to the liquid passage,
in which the vent (150) is disposed in the first portion (116) of the cap (106), the vent (150) being configured to permit displacement of air from inside the housing (102) by any liquids introduced thereto,
in which the vent (150) and the third port (132) are configured to be blocked by the housing (102) when the cap is in a depressed configuration such that the seal is punctured by the tip portion of the insert.

2. The biological indicator (100) of claim 1, in which the sidewall (134) includes a round portion (136) and a flat portion (138).

3. The biological indicator (100) of claim 1 or 2, in which the liquid passage extends alongside the flat portion (138) of the sidewall (134).

4. The biological indicator (100) of any of the preceding claims, in which the cap (106) further comprises a detent (148).

5. The biological indicator (100) of claim 4, in which the detent (148) is disposed in the first portion (118) of the cap (106).

6. The biological indicator (100) of any of the preceding claims, in which the cap (106) is coupled to housing (102) via a friction fit.

7. The biological indicator (100) of claim 6, in which the friction fit comprises contact between an inner surface (142) of the first portion (118) of the cap (106) and an outside surface (114) of the housing (102).

8. The biological indicator (100) of any of the preceding claims, in which the seal (128) comprises a film.

9. The biological indicator (100) of claim 8, in which the film comprises a foil.

10. The biological indicator (100) of any of the preceding claims, in which the tip portion (152) comprises a spike.

11. The biological indicator (100) of claim 10, in which the spike comprises a forked spike.

12. The biological indicator (100) of claim 11, in which the forked spike includes at least two tines (154).

13. The biological indicator (100) of claim 12, in which the at least two tines (154) each includes at least one rib (156).

14. A method of using a biological indicator (100) comprising:
receiving the biological indicator (100), the biological indicator (100) comprising a housing (102) defining a longitudinal axis (104), the housing (102) having a first port (112) and a carrier (110) as source of microorganisms or active enzymes;
a cap (106) coupled to the housing (102), the cap (106) comprising,
a first portion (118) disposed outside the housing (102), the first portion comprising a third port disposed through the first portion, and a second portion (120) disposed at least partially inside the housing (102), the second portion (120) including a liquid chamber (122) comprising a second port (126) and containing a growth medium (124), and a seal (128) covering the second port (126) to define a bottom boundary (130) of the liquid chamber (122), and
an insert (108) disposed in the housing (102), the insert (108) having a tip portion (152) disposed along the longitudinal axis (104);
disposing the biological indicator (100) in a chamber of a decontamination system;
flowing a liquid decontaminant into the housing (102) through a third port (132) in the cap (106); and
removing the liquid decontaminant from the housing (102) through the third port (132) in the cap,
in which a vent (150) is disposed in the first portion (118) of the cap (106), the vent (150) being configured to permit displacement of air from inside the housing (102) by any liquids introduced thereto,
in which the vent (150) and the third port (132) are configured to be blocked by the housing (102) when the cap is in a depressed configuration such that the seal is punctured by the tip portion of the insert.

15. The method of claim 14, in which the step of disposing the biological indicator (100) in the chamber (122) comprises positioning the biological indicator (100) in a substantially horizontal position.

16. The method of claim 14 or 15, in which the step of disposing the biological indicator (100) in the chamber (122) comprises positioning the biological indicator such that the third port (132) faces down.

17. The method of claim 14, 15, or 16, further comprising:
removing the biological indicator (100) from the chamber (122);
depressing the cap (106) relative to the housing (102); and
puncturing the seal (128).

18. The method of claim 17, further comprising submersing a spore disk in the growth medium (124).

## Patentansprüche

1. Biologischer Indikator (100), umfassend:
ein Gehäuse (102), das eine Längsachse (104) definiert, wobei das Gehäuse (102) eine erste Öffnung (112) aufweist;
eine Kappe (106), die mit dem Gehäuse (102) gekoppelt ist, wobei die Kappe (106) umfasst
eine Entlüftungsöffnung (150),
einen ersten Abschnitt (118), der außerhalb des Gehäuses (102) angeordnet ist, wobei der erste Abschnitt eine durch den ersten Abschnitt hindurch angeordnete dritte Öffnung umfasst,
einen zweiten Abschnitt (120), der mindestens teilweise innerhalb des Gehäuses (102) angeordnet ist, wobei der zweite Abschnitt (120) eine Flüssigkeitskammer (122), die eine zweite Öffnung (126) umfasst und ein Wachstumsmedium (124) enthält, und eine Dichtung (128) beinhaltet, die die zweite Öffnung (126) abdeckt, um eine untere Begrenzung (130) der Flüssigkeitskammer (122) zu definieren, und
einen Einsatz (108), der in dem Gehäuse (102) angeordnet ist, wobei der Einsatz (108) einen Spitzenabschnitt (152) aufweist, der entlang der Längsachse (104) angeordnet ist, wobei der zweite Abschnitt (120) der Kappe (106) eine Seitenwand (134) umfasst, die eine seitliche Begrenzung (146) der Flüssigkeitskammer (122) definiert, sodass die zweite Öffnung (126) in dem Gehäuse (102) um die Längsachse (104) des Gehäuses (102) angeordnet ist,
weiter einen Flüssigkeitskanal umfassend, der sich durch einen Raum erstreckt, der zwischen der Seitenwand (134) des zweiten Abschnitts (120) der Kappe (106) und einer Innenfläche (116) des Gehäuses (102) angeordnet ist, wobei die dritte Öffnung (132) Zugang zu dem Flüssigkeitskanal bereitstellt,
wobei die Entlüftungsöffnung (150) in dem ersten Abschnitt (116) der Kappe (106) angeordnet ist, wobei die Entlüftungsöffnung (150) so konfiguriert ist, dass sie das Verdrängen von Luft aus dem Inneren des Gehäuses (102) durch in dieses eingebrachte Flüssigkeiten ermöglicht,
wobei die Entlüftungsöffnung (150) und die dritte Öffnung (132) so konfiguriert sind, dass sie von dem Gehäuse (102) versperrt werden, wenn sich die Kappe in einer eingedrückten Konfiguration befindet, sodass die Dichtung durch den Spitzenabschnitt des Einsatzes durchstochen wird.

2. Biologischer Indikator (100) nach Anspruch 1, wobei die Seitenwand (134) einen runden Abschnitt (136) und einen flachen Abschnitt (138) beinhaltet.

3. Biologischer Indikator (100) nach Anspruch 1 oder 2, wobei sich der Flüssigkeitskanal entlang des flachen Abschnitts (138) der Seitenwand (134) erstreckt.

4. Biologischer Indikator (100) nach einem der vorstehenden Ansprüche, wobei die Kappe (106) weiter eine Rastvorrichtung (148) umfasst.

5. Biologischer Indikator (100) nach Anspruch 4, wobei die Rastvorrichtung (148) in dem ersten Abschnitt (118) der Kappe (106) angeordnet ist.

6. Biologischer Indikator (100) nach einem der vorstehenden Ansprüche, wobei die Kappe (106) über eine Reibpassung mit dem Gehäuse (102) gekoppelt ist.

7. Biologischer Indikator (100) nach Anspruch 6, wobei die Reibpassung Kontakt zwischen einer Innenfläche (142) des ersten Abschnitts (118) der Kappe (106) und einer Außenfläche (114) des Gehäuses (102) umfasst.

8. Biologischer Indikator (100) nach einem der vorstehenden Ansprüche, wobei die Dichtung (128) einen Film umfasst.

9. Biologischer Indikator (100) nach Anspruch 8, wobei der Film eine Folie umfasst.

10. Biologischer Indikator (100) nach einem der vorstehenden Ansprüche, wobei der Spitzenabschnitt (152) einen Dorn umfasst.

11. Biologischer Indikator (100) nach Anspruch 10, wobei der Dorn einen gegabelten Dorn umfasst.

12. Biologischer Indikator (100) nach Anspruch 11, wobei der gegabelte Dorn mindestens zwei Zinken (154) beinhaltet.

13. Biologischer Indikator (100) nach Anspruch 12, wobei die mindestens zwei Zinken (154) jeweils mindestens eine Rippe (156) beinhalten.

14. Verfahren zur Verwendung eines biologischen Indikators (100), umfassend:
Empfangen des biologischen Indikators (100), wobei der biologische Indikator (100) ein Gehäuse (102) umfasst, das eine Längsachse (104) definiert, wobei das Gehäuse (102) eine erste Öffnung (112) und einen Träger (110) als Quelle von Mikroorganismen oder aktiven Enzymen aufweist;
eine Kappe (106), die mit dem Gehäuse (102) gekoppelt ist, wobei die Kappe (106) umfasst
einen ersten Abschnitt (118), der außerhalb des Gehäuses (102) angeordnet ist, wobei der erste Abschnitt eine durch den ersten Abschnitt hindurch angeordnete dritte Öffnung umfasst, und einen zweiten Abschnitt (120), der mindestens teilweise innerhalb des Gehäuses (102) angeordnet ist, wobei der zweite Abschnitt (120) eine Flüssigkeitskammer (122), die eine zweite Öffnung (126) umfasst und ein Wachstumsmedium (124) enthält, und eine Dichtung (128) beinhaltet, die die zweite Öffnung (126) abdeckt, um eine untere Begrenzung (130) der Flüssigkeitskammer (122) zu definieren, und
einen Einsatz (108), der in dem Gehäuse (102) angeordnet ist, wobei der Einsatz (108) einen Spitzenabschnitt (152) aufweist, der entlang der Längsachse (104) angeordnet ist;
Anordnen des biologischen Indikators (100) in einer Kammer eines Dekontaminationssystems;
Einleiten eines flüssigen Dekontaminationsmittels durch eine dritte Öffnung (132) in der Kappe (106) in das Gehäuse (102); und
Entfernen des flüssigen Dekontaminationsmittels aus dem Gehäuse (102) durch die dritte Öffnung (132) in der Kappe,
wobei eine Entlüftungsöffnung (150) in dem ersten Abschnitt (118) der Kappe (106) angeordnet ist, wobei die Entlüftungsöffnung (150) so konfiguriert ist, dass sie das Verdrängen von Luft aus dem Inneren des Gehäuses (102) durch in dieses eingebrachte Flüssigkeiten ermöglicht,
wobei die Entlüftungsöffnung (150) und die dritte Öffnung (132) so konfiguriert sind, dass sie von dem Gehäuse (102) versperrt werden, wenn sich die Kappe in einer eingedrückten Konfiguration befindet, sodass die Dichtung durch den Spitzenabschnitt des Einsatzes durchstochen wird.

15. Verfahren nach Anspruch 14, wobei der Schritt des Anordnens des biologischen Indikators (100) in der Kammer (122) das Positionieren des biologischen Indikators (100) in einer im Wesentlichen horizontalen Position umfasst.

16. Verfahren nach Anspruch 14 oder 15, wobei der Schritt des Anordnens des biologischen Indikators (100) in der Kammer (122) das Positionieren des biologischen Indikators so umfasst, dass die dritte Öffnung (132) nach unten gewandt ist.

17. Verfahren nach Anspruch 14, 15 oder 16, weiter umfassend:
Entfernen des biologischen Indikators (100) aus der Kammer (122);
Niederdrücken der Kappe (106) relativ zu dem Gehäuse (102); und
Durchstechen der Dichtung (128).

18. Verfahren nach Anspruch 17, das weiter das Eintauchen einer Sporenscheibe in das Wachstumsmedium (124) umfasst.

## Revendications

1. Indicateur biologique (100), comprenant :
un boîtier (102) définissant un axe longitudinal (104), le boîtier (102) comportant un premier orifice (112) ;
un bouchon (106) couplé au boîtier (102), le bouchon (106) comprenant :
un orifice de ventilation (150),
une première partie (118) disposée à l'extérieur du boîtier (102), la première partie comprenant un troisième orifice traversant la première partie,
une deuxième partie (120) disposée au moins partiellement à l'intérieur du boîtier (102), la deuxième partie (120) comprenant une chambre à liquide (122) comportant un deuxième orifice (126) et contenant un milieu de culture (124), ainsi qu'un joint d'étanchéité (128) recouvrant le deuxième orifice (126) afin de définir une limite inférieure (130) de la chambre à liquide (122), et
un insert (108) disposé dans le boîtier (102), l'insert (108) comportant une partie d'embout (152) disposée le long de l'axe longitudinal (104), dans lequel la deuxième partie (120) du capuchon (106) comprend une paroi latérale (134) définissant une limite latérale (146) de la chambre à liquide (122) de telle sorte que le deuxième orifice (126) soit disposé dans le boîtier (102) autour de l'axe longitudinal (104) du boîtier (102),
comprenant en outre un passage de liquide qui s'étend à travers un espace situé entre la paroi latérale (134) de la deuxième partie (120) du capuchon (106) et une surface intérieure (116) du boîtier (102), le troisième orifice (132) donnant accès au passage de liquide,
dans lequel l'évent (150) est disposé dans la première partie (116) du bouchon (106), l'évent (150) étant configuré pour permettre l'évacuation de l'air présent à l'intérieur du boîtier (102) par tout liquide qui y est introduit,
dans lequel l'orifice d'aération (150) et le troisième orifice (132) sont agencés de manière à être obstrués par le boîtier (102) lorsque le capuchon est en position enfoncée, de sorte que le joint soit perforé par la partie d'extrémité de l'insert.

2. Indicateur biologique (100) selon la revendication 1, dans lequel la paroi latérale (134) comprend une partie arrondie (136) et une partie plate (138).

3. Indicateur biologique (100) selon la revendication 1 ou 2, dans lequel le passage de liquide s'étend le long de la partie plate (138) de la paroi latérale (134).

4. Indicateur biologique (100) selon l'une quelconque des revendications précédentes, dans lequel le capuchon (106) comprend en outre un cran d'arrêt (148).

5. Indicateur biologique (100) selon la revendication 4, dans lequel le cran d'arrêt (148) est disposé dans la première partie (118) du capuchon (106).

6. Indicateur biologique (100) selon l'une quelconque des revendications précédentes, dans lequel le capuchon (106) est couplé au boîtier (102) par un ajustement par friction.

7. Indicateur biologique (100) selon la revendication 6, dans lequel l'ajustement par friction comprend un contact entre une surface intérieure (142) de la première partie (118) du capuchon (106) et une surface extérieure (114) du boîtier (102).

8. Indicateur biologique (100) selon l'une quelconque des revendications précédentes, dans lequel le joint d'étanchéité (128) comprend un film.

9. Indicateur biologique (100) selon la revendication 8, dans lequel le film comprend une feuille métallique.

10. Indicateur biologique (100) selon l'une quelconque des revendications précédentes, dans lequel la partie d'extrémité (152) comprend une pointe.

11. Indicateur biologique (100) selon la revendication 10, dans lequel la pointe comprend une pointe fourchue.

12. Indicateur biologique (100) selon la revendication 11, dans lequel la pointe fourchue comprend au moins deux dents (154).

13. Indicateur biologique (100) selon la revendication 12, dans lequel les au moins deux dents (154) comprennent chacune au moins une nervure (156).

14. Procédé d'utilisation d'un indicateur biologique (100) comprenant :
la réception de l'indicateur biologique (100), l'indicateur biologique (100) comprenant un boîtier (102) définissant un axe longitudinal (104), le boîtier (102) comportant un premier orifice (112) et un support (110) servant de source de micro-organismes ou d'enzymes actives ;
un capuchon (106) couplé au boîtier (102), le capuchon (106) comprenant :
une première partie (118) disposée à l'extérieur du boîtier (102), la première partie comprenant un troisième orifice traversant la première partie, et une deuxième partie (120) disposée au moins partiellement à l'intérieur du boîtier (102), la deuxième partie (120) comprenant une chambre à liquide (122) comportant un deuxième orifice (126) et contenant un milieu de culture (124), et un élément d'étanchéité e (128) recouvrant le deuxième orifice (126) pour définir une limite inférieure (130) de la chambre à liquide (122), et
un insert (108) disposé dans le boîtier (102), l'insert (108) comportant une partie d'extrémité (152) disposée le long de l'axe longitudinal (104) ;
placer l'indicateur biologique (100) dans une chambre d'un système de décontamination ;
faire s'écouler un liquide décontaminant dans le boîtier (102) par un troisième orifice (132) du bouchon (106) ; et
retirer le décontaminant liquide du boîtier (102) par le troisième orifice (132) du bouchon,
dans lequel un orifice d'aération (150) est disposé dans la première partie (118) du capuchon (106), l'orifice d'aération (150) étant configuré pour permettre l'évacuation de l'air présent à l'intérieur du boîtier (102) par tout liquide y étant introduit,
dans lequel l'évent (150) et le troisième orifice (132) sont configurés pour être obstrués par le boîtier (102) lorsque le capuchon est en position enfoncée, de telle sorte que le joint soit perforé par la partie d'extrémité de l'insert.

15. Procédé selon la revendication 14, dans lequel l'étape consistant à disposer l'indicateur biologique (100) dans la chambre (122) comprend le positionnement de l'indicateur biologique (100) dans une position sensiblement horizontale.

16. Procédé selon la revendication 14 ou 15, dans lequel l'étape consistant à placer l'indicateur biologique (100) dans la chambre (122) comprend le positionnement de l'indicateur biologique de telle sorte que le troisième orifice (132) soit orienté vers le bas.

17. Procédé selon la revendication 14, 15 ou 16, comprenant en outre :
le retrait de l'indicateur biologique (100) de la chambre (122) ;
l'enfoncement du capuchon (106) par rapport au boîtier (102) ; et
percer le joint d'étanchéité (128).

18. Procédé selon la revendication 17, comprenant en outre l'immersion d'un disque de spores dans le milieu de culture (124).
